## Europäisches Patentamt
## European Patent Office
## Office européen des brevets

(19)

(11) Numéro de publication: **0 082 080**
**B1**

(12) # FASCICULE DE BREVET EUROPEEN

(45) Date de publication du fascicule du brevet:
**19.03.86**

(51) Int. Cl.⁴: **G 08 C 19/00,** E 21 F 17/18,
G 01 N 33/00

(21) Numéro de dépôt: **82402275.0**

(22) Date de dépôt: **14.12.82**

(54) **Procédé et dispositif de télétransmission de signaux et application à la détection et/ou la mesure de la teneur en gaz combustible d'une atmosphère.**

(30) Priorité: **14.12.81 FR 8123272**

(43) Date de publication de la demande:
**22.06.83 Bulletin 83/25**

(45) Mention de la délivrance du brevet:
**19.03.86 Bulletin 86/12**

(84) Etats contractants désignés:
**AT BE CH DE FR GB IT LI LU NL SE**

(56) Documents cités:
**DE - A - 2 428 173**
**FR - A - 2 350 461**
**FR - A - 2 427 466**
**US - A - 4 007 458**
**US - A - 4 198 621**
**US - A - 4 222 035**

(73) Titulaire: **Etablissement public dit: CHARBONNAGES DE FRANCE, 9, Avenue Percier, F-75008 Paris (FR)**

(72) Inventeur: **Boutonnat, Maurice, Route des Carrières, F-60270 Gouvieux (FR)**
Inventeur: **Rose, Gérard, 100, rue A. Briand, F-60470 Villers Saint Paul (FR)**

(74) Mandataire: **Rinuy, Santarelli, 14, avenue de la Grande Armée, F-75017 Paris (FR)**

## Description

La présente invention concerne un procédé et un dispositif de télétransmission de signaux et leur application à la détection et/oula mesure de la teneur en gaz combustible d'une atmosphère.

Il est connu de transmettre de l'énergie à un capteur de signal de

mesure à l'aide d'une ligne qui, simultanément, assure la transmission d'une part des signaux d'interrogation du capteur et d'autre part des signaux d'information émis par le capteur. Ainsi le brevet français n° 2.350.461 décrit un procédé et un dispositif de télétransmission dans lesquels l'alimentation en énergie des capteurs est réalisée à l'aide de courant continu véhiculé par la ligne reliant le ou les capteurs à l'unité centrale de traitement et dans lesquels le procédé d'interrogation des capteurs consiste à envoyer sur ladite ligne un courant également continu mais de polarité inverse de celui servant à alimenter les capteurs en énergie.Si l'on désire alimenter à l'aide d'une ligne une multiplicité de capteurson se heurte, compte tenu par exemple de la consommation intrinsèque en énergie des capteurs et d'impératifs de sécurité, à l'impossibilité d'alimenter la ligne en courant continu.

Un premier objet de l'invention concerne un procédé de télétransmission de signaux délivrés par au moins un capteur alimenté en énergie et relié à une ligne de télétransmission, ladite ligne remplissant la triple fonction de fourniture d'énergie audit capteur, de transmission des signaux d'interrogation audit capteur et de transmission des signaux d'information provenant dudit capteur, caractérisé en ce que l'énergie nécessaire à l'alimentation dudit capteur est transmise par la ligne sous forme de courant alternatif d'intensité efficace sensiblement constante ence que les signaux d'interrogation et d'information sont transmis sur la ligne sous la forme numérique, et en ce que chaque signal numérique est transmis sur une période complète du courant alternatif d'alimentation de la ligne.

La ligne de télétransmission peut en outre transmettre des signaux d'interrogation à, et des signaux d'information de, au moins un capteur non alimenté en énergie par ladite ligne.

Ce procédé permet, grâce à la présence de transformateurs adéquats, d'alimenter les capteurs (plus particulièrement d'assurer la charge des batteries d'accumulateurs qui assurent leur fonctionnement) à l'aide d'un courant d'intensité suffisante tout en ne faisant supporter àla ligne qu'un courant d'intensité plus faible (par exemple quelques milliampères).

Ce procédé permet en outre de pouvoir utiliser directement le courant alternatif d'alimentation de la ligne, pour la transmission des signaux d'interrogation et d'information au lieu d'un courant continu.

Avantageusement, les courants circulant dans la ligne conservent une phase constante à $\pi$ près.

La ligne étant alimentée par un courant alternatif d'intensité efficace sensiblement constante quelles que soient l'impédance et la longueur de la ligne, une variation de la résistance de charge au secondaire du transformateur monté en amont du capteur se traduira par une variation de la tension aux bornes dudit transformateur. Cette variation de tension sera transmise intégralement, du fait de la constance des courants, à l'autre extrémité de la ligne. La même valeur de la variation de tension sera obtenue à une extrémité et à l'autre de la ligne. On peut donc se servir de cette variation pour réaliser une transmission numérique des signaux d'interroʒation et d'information par l'intermédiaire de la ligne. On transmettra par exemple un bit 0 lorsque la résistance aux bornes du secondaire du transformateur associé au capteur aura une valeur R et un bit 1 lorsque cette résistance aura une valeur R' supérieure à R. La mesure de la tension permettra de reconnaitre un bit 1 d'un bit 0.

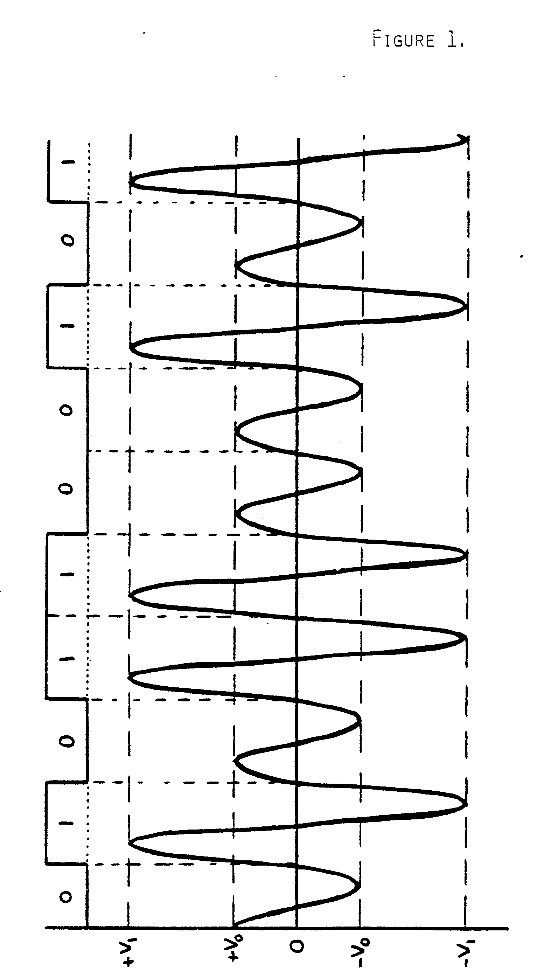

Le message fourni par le capteur interrogé se trouve ainsi acheminé sur la ligne sous la forme représentée schématiquement et par exemple à la figure 1.

Pour éviter de créer des parasites il est nécessaire que la commutation s'effectue aux passages par zéro du courant d'alimentation. Il est, de plus, avantageux, de façon à assurer une bonne fiabilité à latransmission, de choisir de transmettre chaque bit sur une période complète du courant alternatif d'alimentation de la ligne. La synchronisation entre les deux extrémités de la ligne est assurée par le courant alternatif lui-meme. Pendant la durée de la transmission des signaux d'information, il faut déconnecter de la ligne l'accumulateur fournissant l'énergie au capteur et aux circuits qui lui sont le cas échéant associés.

Le procédé de télétransmission selon l'invention peut, en outre, être caractérisé en ce qu'on choisit, comme mode d'interrogation de la ligne, de couper deux périodes consécutives du courant alternatif d'alimentation de ladite ligne.

Le procédé selon l'invention peut en outre être caractérisé en ce que les signaux numériques d'information transmis par la ligne proviennent de la conversion de signaux analogiques non linéaires émis par des détecteurs non linéaires de grandeurs physiques. Pour que la valeur numériquex de la grandeu physique mesurée soit déterminée correctement, on comparele signal analogique non linéaire S émis par le détecteur à une tensionde reference fonction du temps et dont la courbe représentative est constituée de n segments de droite. Cette fonction $V = f(t)$ peut être générée àl'aide de $n + 1$ amplificateurs opérationnels de tension, et a la même allure quela courbe de réponse du détecteur $S = f(x)$. On compare S et V et un dispositif qui comprend une horloge électronique, générant des impulsions proportionnellement au temps t et un compteur comptant lesdits impulsions, dispositif qui est associé au comparateur s'arrete quand la tension de référence V devient égale à la valeur du signal S. Le nombre d'impulsions délivrées au bout du temps t pour lequel V = S est donc une mesure de la valeur x de la grandeur physique, à un coefficient de proportionnalité près, fonctionde la cadence de l'horloge. Cette valeur peut être visualisée et/ou commander un système d'alarme de dépassement de seuil prédéterminé, sur le lieumême où s'effectue la mesure.

Un second objet de la présente invention concerne un dispositif de télétransmission de signaux comprenant au moins une ligne de transmission bifilaire alimentée en courant alternatif et comportant un tranformateur à chacune de ses extrémités, au moins un capteur alimenté en énergie par chaque ligne de transmission, une unité centrale de contrôle et de traitement informatisé, au moins un modulateur-démodulateur relié d'une partà l'unité centrale et d'autre part au transformateur situé à une extrémité de chaque ligne de transmission, et au moins un modulateur-démodulateur relié au transformateur situé à l'autre extrémité de chaque ligne de transmission, chaque capteur étant relié à chaque modulateur-démodulateur relié à ladite autre extrémité de chaque ligne de transmission.

Le dispositif selon l'invention peut comprendre en outre au moins un capteur alimenté en énergie par une source indépendante de chaque ligne de transmission.

On appellera ci-après ""amont" tout ou partie du dispositif situé à proximité de l'unité centrale et "aval" tout ou partie du dispositifsitué à proximité d'un capteur. On appellera "modem" un modulateur-démodulateur.

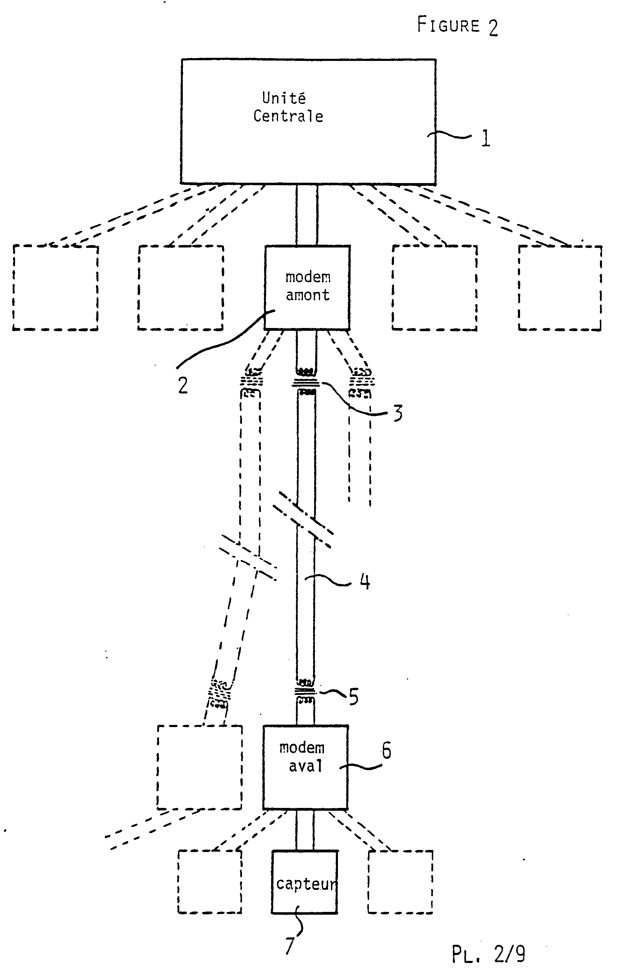

- Un modem aval peut-être connecté au transformateur aval d'une ligne de transmission bifilaire soit par l'intermédiaire de l'enroulement primaire (donc en n'importe quel point de la ligne de transmission situé entre le transformateur aval et le transformateur amont; dans ce cas le transformateur aval peut être intégré au capteur), soit par l'intermédiaire de l'enroulement secondaire du transformateur aval; ledit transformateur aval peut donc être, dans ce second cas, connecté en n'importe quel point d'une ligne situé entre le transformateur amont et le modem aval. Ce second mode de réalisation du dispositif selon l'invention est schématisé àla figure 2 où (1) représente l'unité centrale de contrôle et de traitemeit informatisé, (2) l'un des modems amont, (3) l'un des transformateurs amont (4) l'une des lignes de transmission bifilaire, (5) l'un des transformateurs aval, (6) l'un des modems aval et (7) l'un des capteurs.

Selon un mode de réalisation particulier, un transformateur d'isolement peut, en outre, être intercalé si nécessaire en n'importe quel point d'au moins une ligne.

Dans le cas où le dispositif selon l'invention comprend plusieurs lignes de transmission, les transformateurs amont ont pour fonction de séparer galvaniquement les lignes entre elles; en outre chaque transformateur amont sépare galvaniquement chaque ligne de transmission du modem amont auquel elle est reliée.

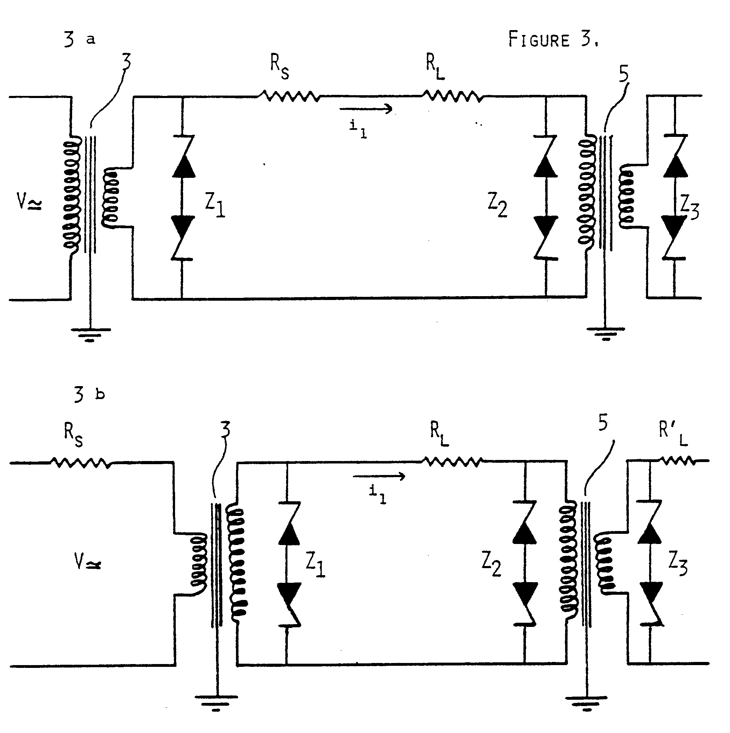

Selon un mode de réalisation les transformateurs amont et aval sont abaisseurs de potentiel; selon un autre mode de réalisation le transformateur amont est élévateur de potentiel et le transformateur aval abaisseur de potentiel. Dans ces deux modes, illustrés respectivement par les figures 3a et 3b, l'intensité $i_1$ du courant véhiculé par la ligne est faible en regard de celui qui alimente le capteur. $Z_1$, $Z_2$, $Z_3$sont des diodes Zener, $R_s$ une résistance additionnelle, $R_L$ est la résistance de la ligne. L'homme de l'art peut aisément, connaissant la tension V, la longueur de la ligne, et donc $R_L$, et les rapports de transformation des transformateurs 3 et 5, en déduire la valeur de $R_s$ de façon à ce quele courant $i_1$ ait la valeur voulue.

A une unité centrale on peut associer un ou plusieurs (par exemple de 1 à 8) modems amont. A chaque modem amont on peut associer une ou plusieurs (par exemple de 1 à 16) lignes de transmission. Chaque ligne est raccordée à un modem aval qui peut lui-même recevoir les informations de un ou plusieurs (par exemple de 1 à 8) capteurs. Ainsi une unité centrale pourra traiter les informations provenant, par exemple, de 8 x 16 x 8 soit 1024 capteurs.

Par capteur on entend un dispositif qui transforme une grandeur physique en un signal analogique (tension ou courant) et convertit le signal analogique en signal numérique. Il comprend donc un détecteur et un convertisseur analogique-numérique. Au moins un des capteurs associés à chaque ligne est alimenté en énergie par ladite ligne. Le modem aval peut en outre et également interroger et recevoir les informations de capteurs ayant une alimentation indépendante de la ligne de transmis sion. Chaque détecteur fournit, lorsque le capteur le comprenant est interrogé par la ligne, un signal analogique, le plus souvent une tension, qui est transformé par le convertisseur analogique-numérique en une information numérique. La liaison entre un capteur et son modem aval est soit une liaison classique (ligne conductrice à 4 fils dont 2 sont réservés à l'interrogation, et 2 à la transmission) soit une liaison opto-électronique assurant une isolation électrique. Dans ce dernier cas le dispositif opto-électronique peut être intégré entièrement soit au modem aval,soit au capteur; il peut aussi être réparti entre le capteur et le modem aval (un transmetteur et un récepteur associés au modem aval, et un transmetteur et un récepteur associés au capteur), la liaison étant alors réalisée à l'aide de fibres optiques. La liaison entre un capteur et son modem aval comprend en outre une ligne bifilaire ayant pour fonction d'alimenter le capteur en énergie.

La conversion analogique-numerique des signaux èmis par le détecteur nécessite:

a) un circuit comprenant une horloge electronique et un compteur d'impuls ions

b) un dispositif capable de générer une fonction V = f (t) composée de n segents de droite et ayant la même forne que la courbe de réponse S = f (x) du detecteur et,

c) un comparateur dont la fonction est de comparer à tout moment la valeur de la tension V = f (t) au signal S = f (x).

Tant que V est inférieure à S, l'horloge fonctionne et fait avancer le compteur. Dès que V = S l'horloge s'arrête ainsi que l'avance du compteur. Les sorties du compteur représentent alors la valeur numérique de la grandeur physique x à mesurer, à un coefficient de proportionnalité près fonction de la cadence de l'horloge.

Le dispositif capable de générer une fonction V = f (t) composée de n segments de droite est, par exemple, constitué de n + 1 amplificateurs opérationnels. Le comparateur peut être un (n + 2)-ième amplificateur opérationnel.

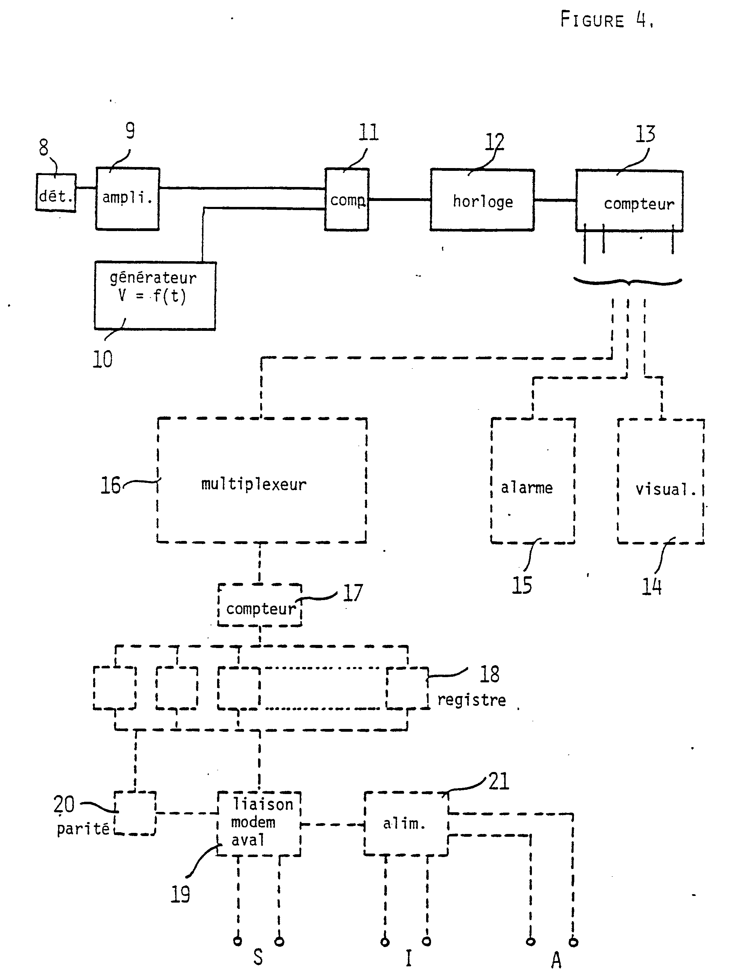

La figure 4 représente schématiquement un cmpteur. Sur cette figure: 8 désigne le detecteur, 9 un amplificateur, 10 le généiateur de fonction V = f (t), 1 1 le comparateur, 12 l'horloge électronique et 13 le compteur d'impulsions. Les éléments suivants peuvent le cas échéant être ajoutés: dispositif de visualisation (14), dispositif de déclenchement d'alarme par dépassement de seuil (15), multiplexeur (16), compteur (17), registre à décalage (18), dispositif pour assurer la liaison avec le modem aval (en partioulier dispositif opto-électronique) (19), cicuit genérateur de bit de parité ou d'imparité (20), dispositiff d'alimentation par la ligne (21).

Le modem aval est alimenté en énergie par un accumulateur rechargeable par la ligne de transmission. Il comprend une horloge électronique dont les impulsions doivent être générées au passage par zéro du courant alternatif véhiculé par la ligne, et une télécommande d'interrogation chargée dans un premier temps de reconnaitre le message d'interrogation et, si ce message est reconnu, d'initialiser un compteur de bits et de réaliser le cycle d'interrogation: séquence d'interrogation du capteur, prise d'informations sur les différents capteurs, envoi des informations sur la ligne.

Le modem amont comporte, si plusieurs lignes lui sont raccordées, deux multiplexeurs qui pernettent de raccorder ce modem à la ligne que l'on veut interroger, un circuit de commande assurant la coupure du courant d'alimentation de la ligne pour une demande d'interrogation ou le renvoi d'un signal d'alarme, un cirouit de détection pour la reconnaissance des informations qui proviennent du modem aval, un circuit d'interface avec l'unité centrale qui effectue le pilotage du modem amont et la saisie de l'information.

L'unité centrale a pour fonction de piloter les modems amont, saisir et traiter les informations (en particulier déclencher les signaux d'alarme, si nécessaire) et, gérer les systèmes périphériques tels que écrans de visualisation, imprimante, etc...

Le procédé et le dispositif selon l'invention trouvent une application partioulièrement intéressante dans la détection et/ou la mesure de la teneur en gaz combustible d'une atmosphère, par exemple dans les galeries de mine, en pernettant de surveiller une multiplicité de zones de travail et donc d'assurer la sécurité dans ces zones.

Dans cette application, le capteur utilisé comprend un détecteur à filament qui, lors de la séquence d'interrogation, est alimenté sous une tension donnée. Le courant parcourant le filament échauffe celui-ci qui, en fonction de la teneur en gaz combustible de l'atmosphère l'environnant, sera porté à une température plus ou moins élevée modifiant sa résistance. Cette modification est utilisée pour générer un déséquilibre (par exemple celui d'un pont de Wheatstone duquel le filament constitue une des branches) et qui sera détecté sous la forme d'une tension constituant le signal analogique de mesure.

Le signal émis par le détecteur n'étant pas linéaire, on le compare à une tension de référence $V = f (t)$ générée par un dispositif, décrit précédemment, comportant n + 1 amplificateurs opérationnels, et associé à un comparateur une horloge électronique et un compteur d'impulsions.

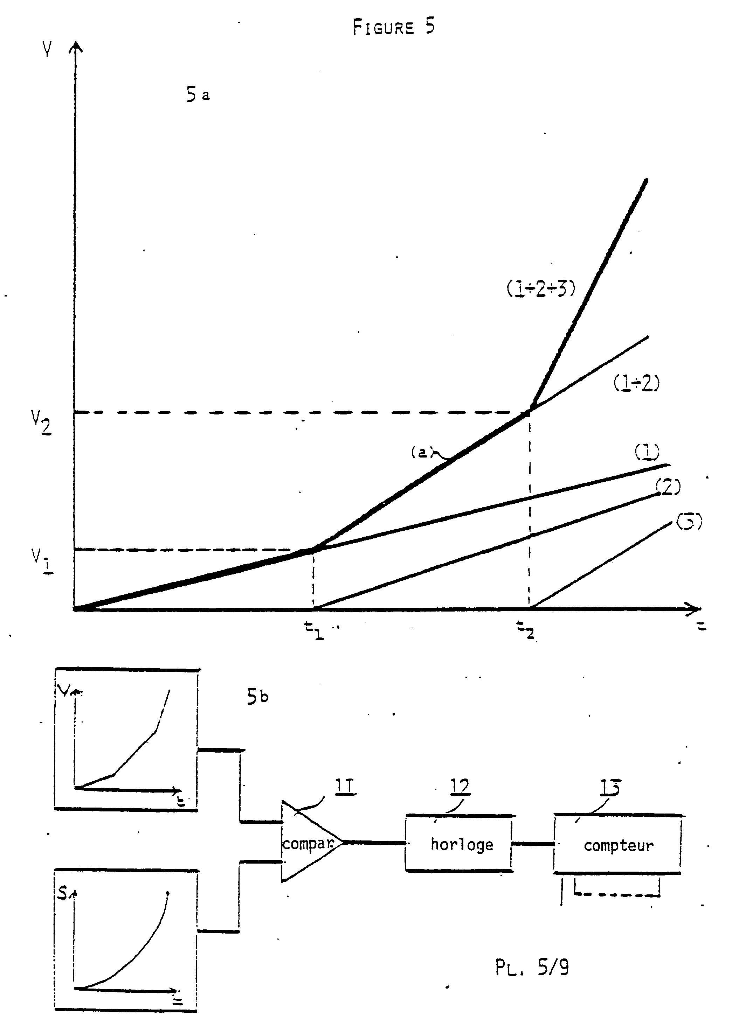

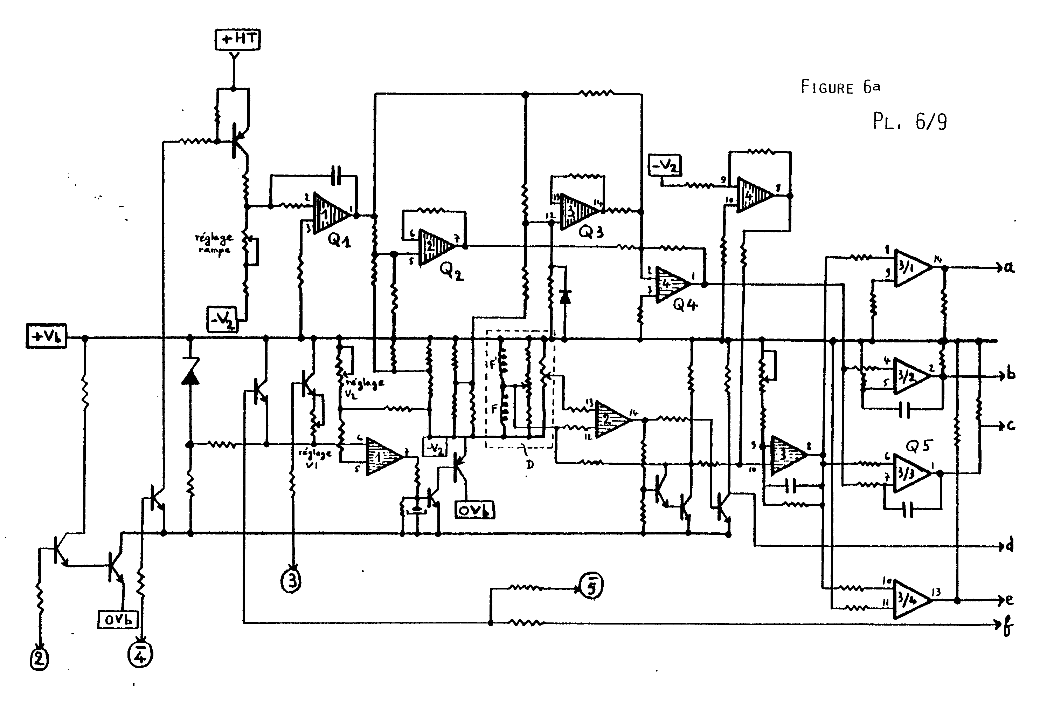

La figure 5a représente la fonction $V = f (t)$ et la figure 5b le dispositif permettant de convertir le paramètre x en une valeur numérique. Cette valeur numérisée en code binaire est ensuite achominée par l'intermédiaire du modem aval, de la ligne de transmission et du modom amont vers l'unité de traitement. Elle peut également être visualisée au niveau même du capteur et également alimenter, sur place, un circuit d'alarme de dépassement de seuil.

Dans cette application particulière du procédé et du dispositif selon l'invention, se pose le problème de la certitude de la mesure. Le résultat d'une interrogation du détecteur dans une atmosphère à forte teneur en gaz combustible (par exemple en méthane) est normalement constitué par l'inversion du signal de mesure délivre par le détecteur. L'emploi de l'inversion du signal délivré par le detecteur ne présente pas une garantie totale quant à la certitude d'une forte teneur en gaz combustible dans l'atmosphère considérée. Plus précisément on a constaté que le phénomène d'inversion du signal de mesure n'est pas conservatif et que certains détecteurs, mêe à l'état neuf, peuvent donner des indications ambigues à cet égard. On a trouvé qu'il est possible d'obtenir qu'un détecteur de teneur en gaz combustible indique sans ambiguïté si la concentration en un tel gaz combustinle dans une atmosphère est ou n'est pas supérieure à un seuil pré-déterminé. Ce résultat est obtenu en interrogeant le détecteur cons écutivement sous deux tens ions d'alimentation différentes. Dans une première étape, du temps 0 au temps $t_1$ de la séquence d'interrogation, on alimente le détecteur sous une première tension $V_1$ et on coupe l'alimentation dès que l'on a détecté un signal positif, et dans une seconde étape, si le signal délivré par le détecteur est négatif ou nul jusqu'au temps $t_1$, on alimente le detecteur, du temps $t_1$ au temps $t_2$ de la séquence d'interrogation, sous une seconde tension $V_2$ supérieureà $V_1$, puis on mesure 1'

élongation du signal au temps $t_2$.

Le dispositif selon l'invention pourra être aménagé, au niveau du capteur, de façon à pourvoir à la double alimentation du détecteur et à la coupure automatique de son alimentation après la phase proprement dite de mesure.

## EXEMPLE - Description et fonctionnement d'un capteur

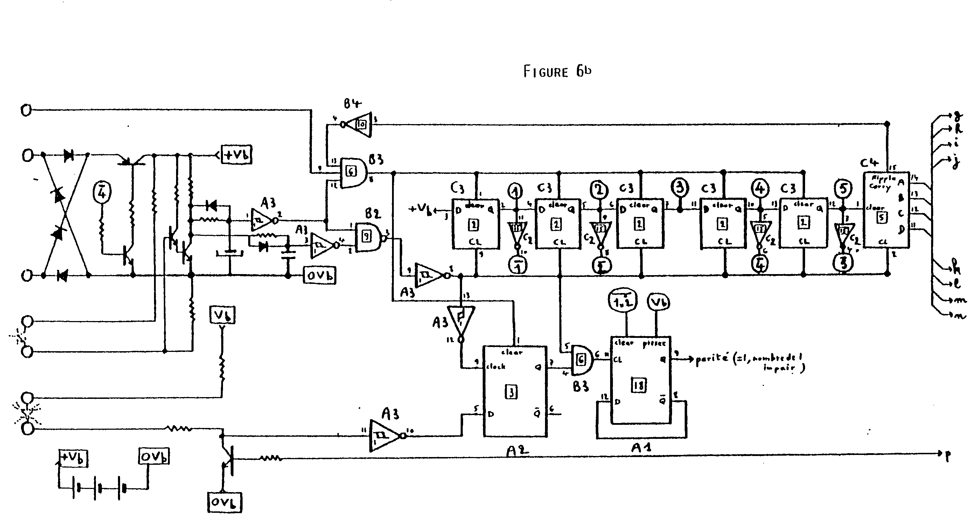

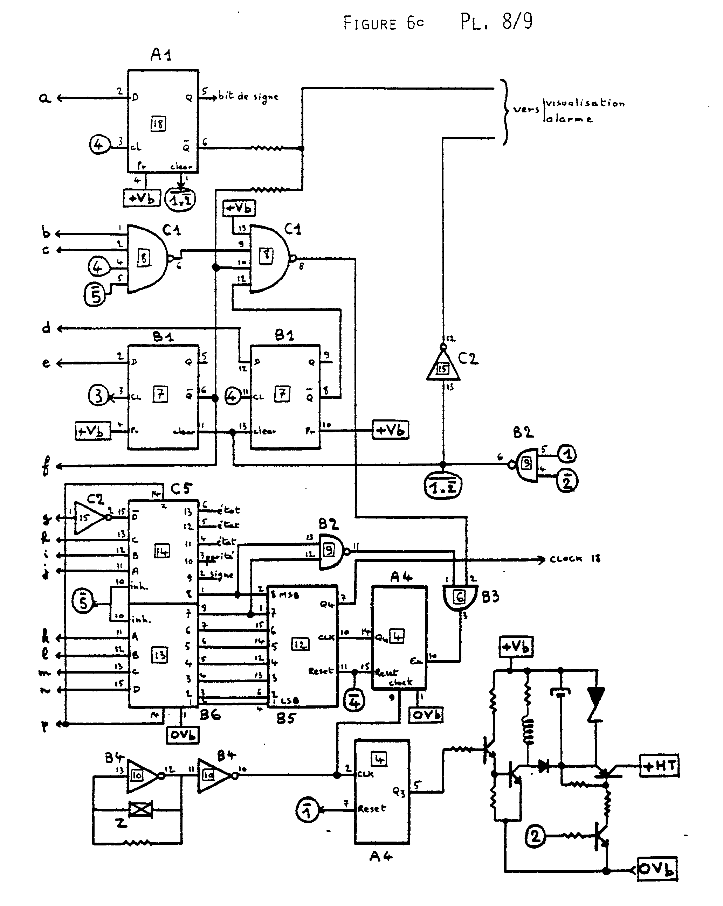

Les figures 6a, 6b et 6c représentent un capteur aménagé pour cette application particulière concernant la mesure de teneurs en gaz combustible dans une atmosphère. Les tensions d'alimentation du détecteur sont élaborées par une source de courant continu rechargeable par la ligne véhiculant du courant à 50 Hz. Sur cette figure les repères ont les significations suivantes:

F est le filament-détecteur, F' le filament-compensateur.

D est le détecteur délivrant la tension du signal de mesure, fonction de la teneur en gaz combustible.

$Q_1$, $Q_2$, $Q_3$, $Q_4$ sont les amplificateurs opérationnels générant la fonction $V = f(t)$.

$Q_5$ est un amplificateur opérationnel remplissant la fonction de comparateur. $A_1$ et $A_2$ sont les ciruits du caloulateur de parite du signal numérique, $A_3$ est un trigger, $A_4$ est le circuit diviseur de l'horloge, $B_1$ est une bascule, $B_2$ et $B_3$ sont deux portes logiques, $B_4$ et Z constituent l'horloge électronique, $B_5$ et $C_4$ sont des compteurs d'impulsions, $B_6$ et $C_5$ sont des multiplexeurs, $C_1$ est un comparateur, $C_2$ est un inverseur logique, et $C_3$ est le registre à décalage.

Le capteur peut comprendre également des afficheurs pour visualisation et une alarme. Ce capteur fonctionne par exemple, à partir du tomps $t = 0$ correspondant à l'envoi au capteur du s ignal d'interrogation par coupure de deux périodes successives du courant d'alimentation, comme suit. Le modem aval ayant reconnu cette coupure envoie une série d'impulsions qui commandent le registre à décalage $C_3$ et le compteur $C_4$. Au temps $t = 50$ ms l'impulsion n° 1 valide la sortie n° 1 (mise en route du convertisseur de tension, remise à zéro des basoules et des afficheurs). Au temps $t = 70$ ms l'impulsion n° 2 valide la sortie n° 2 (alimentation des ciroits analogiques par la batterie et le convertisseur, alimentation du détecteur sous la tension $V_1$; dans le cas de forte teneur en gaz combustible, un signal positif -est détecté et l'alimentation du détecteur est automatiquement coupée). Au temps $t = 670$ ms l'impulsion n° 3 valide la sortie n° 3 (alimentation du détecteur sous la tension $V_2$; l'information recueillie est alors mise en mémoire sur la basoule $B_1$. Sa sortie servira en temps voulu pour envoyer en butée l'indication numérique). Au temps $t = 1970$ ms l'impulsion n° 4 valide la sortie n° 4 (débranchement de la recharge de l'accumulateur, déclenchement de la conversion analogique numérique, validation de la détection d'inversion du signal et du comparateur $C_1$, mise en mèmoire du signe du signal sur la bascule $A_1$). Au temps $t = 2040$ ms l'impulsion n° 5 valide la sortie n° 5 (coupure de l'alimentation du détecteur, fin de validation du compteur $C_1$, validation des multiplexeurs $C_5$-$B_6$ et du compteur $C_4$). Entre le temps $t = 2720$ et 4220 ms les impulsions 6 à 17 valident les sorties de $C_4$, $C_5$ et $B_6$. L'impulsion n° 18 ($t = 4360$ ms) est sans effet. Enfin l'impulsion n° 19 au temps $t = 4380$ $m_s$ a pour effet de couper l'alimentation du capteur et de rebrancher la recharge de l'accumulateur. Un cycle complet d'interrogation dure donc 4400 ms.

## EXEMPLE 2 - Description et fonctionnement d'un modem aval

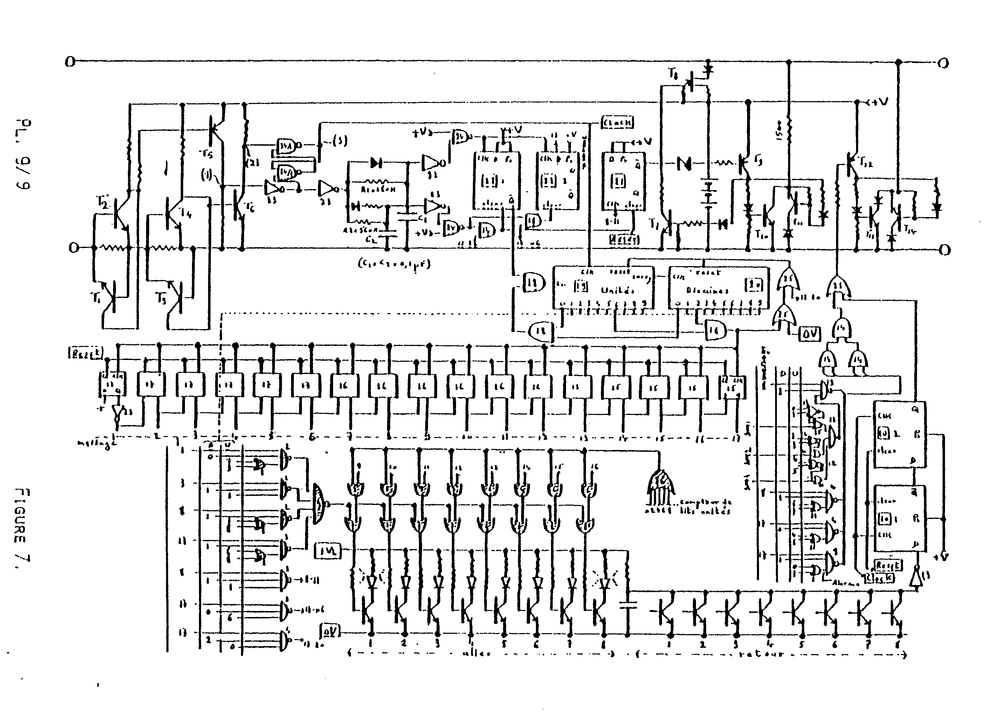

On décrira maintenant de façon plus détaillée un modem aval spécialement destiné à la mise en oeuvre du procédé selon l'invention. Ce modom aval (représenté à la figure 7) est alimenté par un accumulateur rechargé par la ligne véhiculant du courant alternatif à 50 Hz. Les impulsions d'horloge doivent être génerées, corme il a été décritci-dessus, au passage par zéro du courant alternatif. Ces impulsions sont fabriquées par les transistors $T_1$, $T_2$, $T_3$, $T_4$, $T_5$, $T_6$ et les ciroits intégrés 24/1 et 24/2. Lorsque le courant dans la ligne est inférieur à $\pm$ 0,5 mA, on a un signal nul sur le collecteur de $T_6$ (sortie 2). Lorsque le courant dans la ligne est supérieur à $\pm$ 7 mA on a un signal positif sur le collecteur de $T_5$ (sortie 1). Alors la sortie 1 commande le "set" d'une bascule SR (circuits intégrés 24/1 et 24/2), la sortie 2 en commande le "reset", la sortie 3 de la bascule SR délivre les impulsions d'horloge. Ainsi qu'il a été dit ci-dessus le début de la séquence d'interrogation du capteur consiste en une coupure (émise par l'unité centrale) pendant 2 périodes successives du courant alternatif d'alimentation de la ligne. Les constantes de temps des circuits $R_1C_1$ et $R_2C_2$ vérifient que la durée de la coupure est comprise entre 12 et 180 ms. Si la durée est correcte, le compteur de bits (19-20) est initialisé et le modem aval fait son cycle d'interrogation. Dans le cas où la durée est soit inférieure à 12 ms, soit supérieure à 180 ms, par exemple à cause de micro-coupures du courant, d'une ligne coupée accidentellement, de mauvais contacts, le modem aval ne fait pas de cycle d'interrogation et reste transparent.

La transmission du signal par le modem aval sur la ligne se fait après que l'alimentation par la ligne du capteur et du modem ait été automatiquement coupée et qu' une rés istance de 1 500 ohms ait été connectée sur la ligne grâce à l'interrupteur électronique $T_{10}$, $T_{11}$.

Le bit 0 s'obtient en fermant l'interrupteur électronique $T_{13}$, $T_{14}$.

Le bit 1 s'obtient en ouvrant ce même interrupteur.

Le message fourni par le capteur interrogé se trouve ainsi acheminé sur la ligne sous la forme

représentée schèmatiquement et par exemple à la figure 1. Ce message est détecté par le modem amont dont le circuit de détection a été initialisé par un message particulier provenant du modem aval en début de séquence, par exemple un bit 0 puis un bit 1.

Le circuit de détection mesure la tension $2 V_0$ correspondant au bit 0, puis $2 V_1$ correspondant au bit 1. Il en fait ensuite la moyenne:

$$V_m = \frac{2 V_0 + 2 V_1}{2}$$

$= V_0 + V_1$ dont il mettra la valeur en mémoire. La détection se fera ensuite par compàraison à $V_m$:
- si $2 V > V_m$ on décide que le bit est 1.
- si $2 V < V_m$ on décide que le bit est 0.

L' électronique séquentielle du modem aval comprend:

a) une base de temps. Une séquence est divisée en 17 unités de temps ("udt"). Chaque udt dure 13 périodes soit 26 demi-périodes du courant alternatif alimentant la ligne. Les 8 premières udt sont utilisées à l'interrogation des capteurs. Un compteur en base 26 (représenté sur la figure 7 par les compteurs de bits unités et dizaines 19 et 20) compte les passages par zéro du courant de la ligne. Tous les 26 il incrémente le compteur d'unités de temps (circuits intégrés 15, 16 et 17). Un bit quelconque sera envoyé à un moment programmé tenant compte du compteur en base 26 et du compteur d'unités de temps. Par exemple le premier bit SM 1 (premier bit d'état du modem aval) est envoyé en position 20-21 du compteur en base 26 et en position 8 du compteur d'unités de temps,

b) un ensemble de cirouits logiques (représentés avec les barres obliques sur la figure 7) qui, associés aux compteurs, fabrique la séquence d'interrogation des capteurs,

c) les bascules n° 10 qui pernettent de disposer d'un retard suffisamment long (une période) entre le moment où le modem aval demande à un capteur l'envoi d'un bit et le moment où ce modem transmet ledit bit sur la ligne,

d) la basoule n° 22-1 qui met en mémoire la coupure de la ligne et initialise la séquence,

e) la basoule n° 22-2 qui met en mémoire la télécommande d'alarme éventuellement envoyée par l'unité centrale, si elle est apparue à la l7ème unité de temps,

f) la bascule n° 21 chargée, au moment de l'envoi du message sur la ligne, de débrancher l'acommulateur et brancher la résistance de 1500 ohms.

**Revendications**

1. Procédé de télétransmission de signaux delivrés par au moins un capteur (7) alimente en energie et relié à une ligne de télétransmission (4) ladite ligne remplissant la triple fonction de fourniture d'énergie audit capteur, de transmission des signaux d'interrogation audit capteur et de transmission des signaux d'information provenant dudit capteur, caractérisé en ce que l'énergie nécessaire à l'alimentation dudit capteur (7) est transmise par la ligne (4) sous forme de courant alternatif d'intensité efficace sensiblement constante, en ce que les signaux d'interrogation et d'information sont transmis sur la ligne sous la forme numérique, et en ce que chaque signal numérique est transmis sur une période complète du courant alternatif d'alimentation de la ligne.

2. Procédé selon la revendication 1 caractérisé en ce que les courants circulant dans la ligne (4) conservent une phase constante à $\pi$ près.

3. Procédé selon l'une des revendications 1 et 2 caractérisé en ce que la ligne de télétransmission (4) alimente également en énergie les circuits électroniques associés audit capteur (7).

4. Procédé selon l'une des revendications 1 à 3 caractérisé en ce que la ligne de teletransmission (4) transmet en outre des signaux d'interrogation à, et des signaux d'information de, au moins un capteur non alimenté en énergie par ladite ligne.

5. Procédé selon l'une des revendications 1 à 4, caractérisé en ce que le signal numérique d'interrogation de la ligne consiste en une coupure du courant alternatif qui l'alimente pendant deux périodes consécutives.

6. Procédé selon l'une des revendications 1 à 5, caractérisé en ce que les signaux numériques d'information transmis par la ligne (4) proviennent de la conversion de signaux analogiques non linéaires comparés à une tension de référence fonction du temps et dont la courbe représentativeest constituée de n segments de droite.

7. Dispositif de télétransmission de signaux caractérisé en ce qu'il comprend au moins une ligne de transmission bifilaire (4) alimentée en courant alternatif et comportant un transformateur à chacune de ses extrémités, au moins un capteur (7) alimenté en énergie par chaque ligne de transmission, une unité centrale de contrôle et de traitement informatisé (1), au moins un modulateur-démodulateur (2) relié d'une part à l'unité centrale (1) et d'autre part au transformateur (3) situé à une extrémite de chaque ligne de transmission, et au moins un modulateur-démodulateur (6) relié au transformateur (5) situé à l'autre extrémité de chaque ligne de transmission (4), et en ce que chaque capteur (7) est relié à chaque modulateur-démodulateur (6) relié à ladite autre extrémité de chaque ligne de transmission.

8. Dispositif selon la revendication 7, caractérisé en ce qu'il comprend en outre au moins un capteur alimenté en énergie par une sourceindépendante de chaque ligne de transmission.

9. Dispositif selon l'une des revendications 7 et 8, caractérisé en ce que le primaire du transformateur (5) situé à ladite autre extrémité de chaque ligne de transmission (4) est relié au modulateur-démodulateur (6)af-fecté à cette extrémité de chaque ligne.

10. Dispositif selon l'une des revendications 7 et 8, caractérisé en ce que le secondaire du transformateur (5) situé à ladite autre extrémité de chaque ligne de transmission (4) est relié au modulateur-démodulateur(6) affecté à cette extrémité de chaque ligne.

11. Dispositif selon l'une des revendications 7 à 10, caractérisé en ce qu'un transformateur d'isolement est intercalé en n'importe quel point d'au moins une ligne.

12. Dispositif selon l'une des revendications 7 à 11, caractérisé en ce que la liaison entre au moins un capteur (7) et le modulateur-démodulateur (6) relié à ladite autre extrémité de chaque ligne de transmission (4) est constituée par des dispositifs opto-électroniques.

13. Dispositif selon l'une des revendications 7 à 12 caractérisé en ce que le modulateur-démodulateur (6) relié au transformateur (5) situéà ladite autre extrémité de chaque ligne de transmission (4) comprend un accumulateur rechargeable par la ligne, un circuit d'horloge générant des impulsions au passage par zéro du courant alternatif véhiculé par la ligne,un compteur de bits, une base de temps, un ensemble de circuits logiques fabriquant la séquence d'interrogation des capteurs (7) reliés au modulateurdémodulateur (6), deux bascules permettant de disposer d'un retard suffisant entre la réception d'un bit provenant d'un capteur (7) et l'émission de ce bit sur la ligne (4), une bascule ayant pour fonction, au moment de l'envoi du message sur la ligne, de débrancher l'accumulateur et fermer la ligne sur une résistance, un interrupteur électronique ayant pour fonction, par son ouverture et sa fermeture, de transmettre le message sur la ligne.

14. Dispositif selon la revendication 13 caractérisé en ce que le modulateur-démodulateur (6) comprend, en outre, un dispositif opto-électronique pour l'envoi du signal d'interrogation aux capteurs (7)auxquels il est associé et un dispositif opto-électronique pour la réception des signaux d'information provenant desdits capteurs (7).

15. Application du procédé selon l'une des revendications 1 à 6 à la détection et/ou la mesure de la teneur en gaz combustible d'une atmosphère.

**Patentansprüche**

1. Verfahren zur Fernübertragung von Signalen,die von mindestens einem Fühler (7) geliefert werden, der mit Energie gespeist und angeschlossen ist an eine Übertragungsleitung (4) wobei die Leitung die dreifache Funktion der Energieversorgung des Fühlers, der Übertragung von Abfragesignalen zu dem Fühler und der Übertragung von Informationssignalen, die von dem Fühler erzeugt werden, erfüllt, dadurch gekennzeichnet, daß die zur Speisung des Fühlers (7) notwendige Energie durch die Leitung (4) in Form von Wechselstrom mit im wesentlichen konstanter Effektivstromstärke übertragen wird, und daß die Abfrage- und die Informationssignale an der Leitung in digitaler Form übertragen werden, und daß jedes digitale Signal während einer vollständigen Periode des Speise-Wechselstromes an der Leitung übertragen wird.

2. Verfahren nach Anspruch 1, dadurch gekennzeichnet, daß die in der Leitung (4) umlaufenden Ströme eine konstante Phase von nahezu π beibehalten.

3. Verfahren nach einem der Ansprüche 1 und 2, dadurch gekennzeichnet, daß die Fernübertragungsleitung (4) gleichermaßen die dem Fühler (7) zugeordneten elektronischen Schaltungen mit Energie versorgt.

4. Verfahren nach einem der Ansprüche 1 bis 3, dadurch gekennzeichnet, daß die Fernübertragungsleitung (4) außerdem Abfragesignale zu und Informationssignale von mindestens einem nicht durch diese Leitung mit Energie versorgten Fühler überträgt.

5. Verfahren nach einem der Ansprüche 1 bis 4, dadurch gekennzeichnet, daß das digitale Abfragesignal der Leitung in einer Unterbrechung des Speise-Wechselstromes während zwei aufeinanderfolgenden Perioden besteht.

6. Verfahren nach einem der Ansprüche 1 bis 5, dadurch gekennzeichnet, daß die über die Leitung (4) übertragenen digitalen Informationssignale aus der Wandlung von nichtlinearen Analogsignalen im Vergleich mit einer Referenzspannung als Funktion der Zeit entstehen, deren repräsentative Kurve aus n geradlinigen Segmenten gebildet ist.

7. Einrichtung zur Fernübertragung von Signalen, dadurch gekennzeichnet, daß sie mindestens eine zweidrähtige, mit Wechselstrom gespeiste Übertragungsleitung (4) umfaßt und einen Transformator an jedem ihrer Enden enthält, mindestens einen durch die Übertragungsleitung energie-gespeisten Fühler (7) eine zentrale Steuer- und Informationsverarbeitungseinheit (1) mindestens einen einerseits mit der Zentraleinheit (1) und andererseits mit einem an einem Ende der Übertragungsleitung angeordneten Transformator (3) verbundenen Modem (Modulator und Demodulator) (2) und mindestens einen mit einem an dem anderen Ende der Übertragungsleitung (4) angeschlossenen Transformator (5) verbundenen Modem (6) aufweist, und daß jeder Fühler (7) mit jedem an dem anderen Ende der Übertragungsleitung angeschlossenen Modem (6) verbunden ist.

8. Einrichtung nach Anspruch 7, dadurch gekennzeichnet, daß sie außerdem mindestens einen Fühler enthält, der durch eine von der Übertragungsleitung unabhängige Energiequelle gespeist ist.

9. Vorrichtung nach einem der Ansprüche 7 und 8, dadurch gekennzeichnet, daß die Primärseite des an dem anderen Ende jeder Übertragungsleitung (4) angeordneten Transformators (5) mitdem-an-diesem Ende jeder Leitung eingesetzten Modem (6) verbunden ist.

10. Einrichtung nach einem der Ansprüche 7 und 8, dadurch gekennzeichnet, daß die Sekundärseite des an dem anderen Ende jeder Übertragungsleitung (4) angeordneten Transformators (5) mit dem an diesem Ende jeder Leitung eingesetzten Modem (6) verbunden ist.

11. Einrichtung nach einem der Ansprüche 7 bis 10, dadurch gekennzeichnet, daß ein Trenn-Transformator an irgendeiner Stelle mindestens einer Leitung zwischengeschaltet ist.

12. Einrichtung nach einem der Ansprüche 7 bis 11, dadurch gekennzeichnet, daß die Verbindung zwischen mindestens einem Fühler (7) und dem an dem anderen Ende jeder Übertragungsleitung (4) angeschlossenen Modem (6) durch opto-elektronische Einrichtungen gebildet ist.

13. Einrichtung nach einem der Ansprüche 7 bis 12, dadurch gekennzeichnet, daß der mit dem an dem anderen Ende jeder Übertragungsleitung (4) eingesetzten Transformator (5) verbundene Modem (6) einen durch die Leitung wiederaufladbaren Akkumulator enthält, einen Taktkreis, der beim Nulldurchgang des durch die Leitung übertragenen Wechselstromes Impulse erzeugt, einen bit-Zähler, eine Zeitbasis, eine Anordnung von Logikschaltungen, die die Sequenz der Abfrage der mit dem Modem (6) verbundenen Fühler (7) erzeugt, zwei Kippkreise, die das Einsetzen einer ausreichenden Verzögerung zwischen dem Empfang eines von einem Fühler (7) stammenden bit und der Aussendung des bit über die Leitung (4) erlauben, einen Kippkreis, der im Moment der Sendung einer Nachricht über die Leitung das Abschalten des Akkumulators und das Abschließen der Leitung durch einen Widerstand bewirkt, und einen elektronischen Schalter, der durch sein Öffnen und sein Schließen das Aussenden der Nachricht über die Leitung bewirkt.

14. Einrichtung nach Anspruch 13, dadurch gekennzeichnet, daß der Modem (6) außerdem eine opto-elektronische Einrichtung zur Sendung des Abfragesignales an die Fühler (7) enthält, welchen er zugeordnet ist, und eine opto-elektronische Einrichtung zum Empfang der von den Fühlern (7) stammenden Informationssignale.

15. Anwendung des Verfahrens nach einem der Ansprüche 1 bis 6 zur Erfassung und/oder der Messung des Gehaltes an brennbarem Gas in einer Atmosphäre.

**Claims**

1. A method of long distance transmission of signals supplied by at least one sensor (7) fed with energy and connected to a long distance transmission line (4), said line fulfilling the tripple function of energy supply to said sensor, of transmission of interrogation signals to said sensor and of transmission of information signals from said sensor, characterized in that the energy required for feeding said sensor (7) is transmitted by the line (4) as alternative current of substantially constant rms value, and in that the interrogation and information signals are transmitted on the line in digital form, and in that each digital signal is transmitted over a complete cycle ofthe alternative current feeding the line.

2. A method according to claim i, characterized in that the currents flowing in line (4) keep a constant phase except for $\pi$.

3. A method according to one of claims 1 and 2, characterized in that the long distance transmission line (4) also supplies energy to the electronic circuits associated with said sensor (7).

4. A method according to one of claims 1 to 8, characterized in that the long distance transmission line (4) also transmits interrogation signals to, and information signals from, at least one sensor not supplied with energy from said line.

5. A method according to one of claims 1 to 4, characterized in that the interrogation digital signal from the line consists in cutting off the alternative current supplied thereto for two consecutive cycles.

6. A method according to one of claims 1 and 5, characterized in that the digital information signals transmitted by line (4) result from conversion of non linear analog signals compared to a reference voltage as a function of time and the representative curve of which consists of n segments of straight line.

7. A device for long distance transmission of signals, characterized in that it comprises at least one bifilar transmission line (4) supplied with alternative current and comprising a transformer at each of its ends, at least one sensor (7) supplied with energy from each transmission line, a central control and information processing unit (1), at least one modulator-demodulator (2) connected to the central unit (1) on the one hand, and on the other hand, to the transformer (3) located at one end of each transmission line, and at least one modulator-demodulator (6) connected to the transformer (5) located at the other end of each transmission line (4), and in that each sensor (7) is connected to each modulator-demodulator (6) connected to said other end of each transmission line.

8. A device according to claim 7, characterized in that it also comprises at least one sensor fed with energy from a source independent of each transmission line.

9. A device according to one of claims 7 and 8, characterized in that the primary winding of transformer (5) located at said other end of each transmission line (4) is connected to the modulator-demodulator (6) allotted to that end of each line.

10. A device according to one of claims 7 and 8, characterized in that the secondary winding of transformer (5) located at said other end of each transmission line (4) is connected to the modulator-demodulator (6) allotted to that end of each line.

11. A device according to one of claims 7 to 10, characterized in that an insulation transformer is intercalated in any point of at least one line.

12. A device according to one of claims 7 to 11, characterized in that the connection between at least one sensor (7) and the modulator-demodulator (6) connected to said other end of each transmission line (4) is constituted by opto-electronic devices.

13. A device according to one of claims 7 to 12, characterized in that the modulator-demodulator (6) connected to transformer (5) located at said other end of each transmission line (4) comprises a storage cell rechargeable through the line, a clock circuit generating pulses at the zero transition of the alternative current flowing in the line, a bit counter, a time base, a logic circuit arrangement producing the interrogation sequence of the sensors (7) connected to the modulator-demodulator (6), two flip-flops for providing a sufficient delay between reception of a bit from one sensor (7) and transmission of such bit on the line (4), with one flip-flop having the function at the time of sending the message to the line of switching off the storage cell and closing the line on a resistance, an electronic switch having the functionof transmitting the message to the line by the opening and closing thereof.

14. A device according to claim 13, characterized in that the modulator-demodulator (6) also comprises an opto-electronic device for forwarding the interrogation signals to the sensors (7) with which it is associated and an opto-electronic device for receiving information signals from said sensors (7).

15. The application of the method according to one of claims 1 to 6, to detection and/or measurement of the combustible gas concentration in an atmosphere.

0 082 080

FIGURE 1.

PL. 1/9

FIGURE 2

PL. 2/9

FIGURE 3.

PL. 3/9

FIGURE 4.

PL. 4/9

FIGURE 5

5 a

5 b

PL. 5/9

Figure 6a

PL. 6/9

FIGURE 6a

FIGURE 6b

Figure 6c PL. 8/9

0 082 080

15

PL. 9/ 9

FIGURE 7.